# EUROPÄISCHE PATENTSCHRIFT

(11) **EP 3 308 760 B1**
(45) Veröffentlichungstag und Bekanntmachung des Hinweises auf die Patenterteilung: **04.09.2024**
(21) Anmeldenummer: 17198606.0
(22) Anmeldetag: 27.04.2010
(51) Int. Cl.: A61F 13/00, A61F 13/02, A61M 1/00, A61F 13/0206, A61F 13/0203, A61F 13/05

(54) **WUNDABDECKUNG UND VERFAHREN ZUR HERSTELLUNG**
WOUND COVERING AND PROCESS FOR ITS MANUFACTURING
PANSEMENT POUR PLAIE ET T SON PROCÉDÉ DE FABRICATION

(30) Priorität: 30.04.2009 DE 102009019646
(43) Veröffentlichungstag der Anmeldung: 18.04.2018
(62) Teilanmeldung aus: 10716300.8
(73) Patentinhaber: Lohmann & Rauscher GmbH, 2525 Schönau/Triesting (AT)
(72) Erfinder: Wild, Thomas, 1200 Wien (AT); Wagner, Georg, 4040 Linz (AT); Rohrer, Christian, 4020 Linz (AT); Steinlechner, Erik, 2500 Baden (AT)
(74) Vertreter: Herrmann, Daniel

(56) Entgegenhaltungen:
- EP-B1- 2 309 961
- DE-A1- 102006 017 194
- DE-A1- 102007 049 428
- DE-A1- 102008 034 362
- DE-B4- 102006 017 194
- DE-U1- 202006 015 547
- GB-A- 2 085 305
- US-A- 3 929 135
- US-A- 4 341 217
- US-A1- 2004 030 304

## Beschreibung

Die Erfindung betrifft eine Wundabdeckung mit einem eine erste Begrenzungsfläche bildenden ersten bahnförmigen Element und einem eine der ersten Begrenzungsfläche abgewandte und etwa parallel dazu verlaufende zweite Begrenzungsfläche bildenden und mit dem ersten bahnförmigen Element verbundenen zweiten bahnförmigen Element und ein Verfahren zum Herstellen erfindungsgemäßer Wundabdeckungen.

Derartige Wundabdeckungen werden bei verschiedenartigen Krankheitsbildern bzw. Traumata, insbes. im Bereich des offenen Abdomens benötigt, wenn eine temporäre Abdeckung der offenen Wunde bzw. des offenen Abdomens notwendig ist. Das kann bspw. notwendig sein, wenn mehrere Eingriffe täglich erforderlich sind, um einerseits einen schnellen Zugang zu den inneren Organen zu ermöglichen und andererseits einen nachteilhaften Einfluß der Exsudatbildung im Wundbereich zu vermindern. Durch eine temporäre Abdeckung kann ein deutlicher Rückgang der Mortalität bei einigen Indikationen erreicht werden.

Als für die genannten Einsatzzwecke geeignete Wundabdeckungen werden grundsätzlich aus medizinischer Sicht zwei Hauptanforderungen gestellt. Zum einen muß ein gutes Exsudatmanagement im Wundbereich, insbes. bei Einsatz im offenen Abdomen, erreicht werden, d. h. eine Absaugung im gesamten Wundbereich bzw. im gesamten Abdomen. Darüber hinaus sollte eine Friktionsminderung zwischen Organen und Bauchfell und der Wundabdeckung erreicht werden, während gleichzeitig die offene Wunde von der Umwelt gut abgeschirmt ist. Darüber hinaus muß sichergestellt werden, daß durch die Wundabdeckung keine Verunreinigungen in die offene Wunde bzw. den offenen Abdominalbereich gelangen.

In der EP 0 261 167 B1 ist eine flüssigkeitsdurchlässige und für den direkten Kontakt mit dem Wundgrund vorgesehene Wundabdeckung beschrieben, welche eine hydrophobe Schicht aufweist, um das Anhaften der Wundabdeckung am Wundbereich und eine dadurch ggf. verursachte Verunreinigung der Wunde zu verhindern. Mit der in dieser Schrift beschriebenen Wundabdeckung kann jedoch kein zufriedenstellendes Exsudatmanagement im Wundbereich erhalten werden.

Zur Verbesserung des Exsudatmanagements im Wundbereich wird in der US 7,381,859 B2 eine Wundabdeckung vorgeschlagen, bei der zwischen zwei flüssigkeitsdurchlässigen, folienartigen, bahnförmigen Elementen, die in Form von Kunststofffilmen ausgeführt sein können, eine schaumartige Schicht aufgenommen ist, in der Exsudate absorbiert werden können. Bei der aus dieser Schrift bekannten Wundabdeckung hat es sich jedoch als problematisch erwiesen, daß in Randbereichen der Wundabdeckung keine zufriedenstellende Absaugung von Exsudaten erfolgen kann, so daß es in diesen Randbereichen zu Komplikationen bei der Wundversorgung kommt.

In der WO 2007/118652 A1 ist ein Wunddistanzgitter beschrieben, auf das saugfähige Sekundärverbände kleblos auflegbar sind und welches zur Bildung einer ersten glatten Oberfläche und einer zweiten Oberfläche mit einem rauhen Griff mit einer Vielzahl von dreidimensionalen Perforationen ausgestattet ist. Bei Einsatz dieser bekannten Wundabdeckung sind die nachgeschalteten Absorptionskörper auswechselbar, um so über einen längeren Zeitraum eine zufriedenstellende Wundversorgung zu gewährleisten. Allerdings hat es sich auch bei Einsatz der in der genannten Schrift beschriebenen Wundabdeckungssysteme als problematisch erwiesen, daß ein zufriedenstellendes Exsudatmanagement über den gesamten Wundbereich, insbes. im Abdominalbereich, nicht erreichbar ist.

In der US 4,341,217 ist eine Binde mit einem in einer äußeren Hülle aufgenommenen Absorptionskörper angegeben, bei dem die äußere Hülle kanalförmige Öffnungen aufweist. In der DE 10 2006 017 194 ist ein Primärverband zur Wundabdeckung unter Vermeidung von Verklebungen und Adhäsion mit dem Wundexsudat mit einer Vielzahl von dreidimensionalen Perforationen beschrieben.

In der DE 20 2006 015 547 U1 ist ein Verband zur Umlage um eine chirurgisch angelegte Leitung aus einer menschlichen oder tierischen Körperöffnung beschrieben, die wenigstens einen Schlitz aufweist, der es ermöglicht, den Verband am Körper des Patienten um die jeweilige Leitung umzulegen.

In der DE 10 2007 049 428 A1 ist ein Wundpflegeartikel mit einer superabsorbierende Polymere aufweisenden Lage beschrieben, die in einer aus Polyesterfolie gefertigten Innenhülle untergebracht sein kann.

In der US 2004/0030304 A1 ist ein System und ein Verfahren zum temporären Verschließen einer Wunde, insbesondere einer Abdominalwunde, beschrieben, bei dem ein Verband zum Einsatz kommt, der eine Lage aus einem porösen Schaum umfasst, welche von Lagen aus einem elastomeren Material eingeschlossen ist, die von einer Anzahl geeignet angeordneter Löcher durchsetzt sind.

Wundabdeckungen nach dem Oberbegriff des Patentanspruchs 1 sind in der DE 10 2008 034 362 A1 angegeben.

Angesichts der vorstehend beschriebenen Probleme im Stand der Technik liegt der Erfindung die Aufgabe zugrunde, eine Wundabdeckung bereitzustellen, mit der ohne Verunreinigung der Wunde selbst ein gutes Exsudatmanagement über den gesamten Wundbereich möglich ist.

Erfindungsgemäß wird diese Aufgabe durch die im Patentanspruch 1 angegebene Weiterbildung der bekannten Wundabdeckungen gelöst.

Bei der erfindungsgemäßen Wundabdeckung wird durch die Bildung des Drainageraums eine kapillare Wirkung zwischen den einzelnen bahnförmigen Elementen erreicht, mit der einerseits ein Abfließen von Körperflüssigkeiten, insbes. Exsudaten, aus dem Drainageraum verhindert und andererseits eine Verteilung der Körperflüssigkeiten über die gesamte Drainagefläche möglich ist, ohne daß dazu zusätzliche Maßnahmen, wie etwa die Bereitstellung zusätzlicher Absorptionskörper, zwingend erforderlich sind. Auf diese Weise wird ein gutes Exsudatmanagement über den gesamten Wundbereich ermöglicht, weil auch keine das Exsudatmanagement nachteilhaft beeinflussenden Randbereiche ohne Kapillarfunktion erforderlich sind. Die erfindungsgemäße Wundabdeckung läßt sich problemfrei auf jede Größe bzw. jede Struktur von Wunden, im besonderen im Bauchraum, zuschneiden und gewährleistet über die gesamte Wundabdeckungsfläche die gewünschte Drainagefunktion. Dabei kann die Wundabdeckung auch mit einer glatten Oberfläche ausgeführt sein, die ein Anhaften von Geweben und Zellwundgrund bzw. bei den umliegenden Organen verhindert. Der Eintritt von Körperflüssigkeiten in den Drainageraum kann bei Einsatz erfindungsgemäßer Wundabdeckungen bereits dadurch sichergestellt werden, daß die bahnförmigen Elemente selbst aus einem flüssigkeitsdurchlässigen Material gebildet sind.

Im Rahmen der Erfindung ist vorgesehen, wie unter anderem in Anspruch 1 definiert, dass mindestens eines der bahnförmigen Elemente eine den Durchtritt von Körperflüssigkeiten in den Drainageraum ermöglichende Öffnung aufweist. Dabei wird die Stabilität der Gesamtstruktur erfindungsgemäßer Wundabdeckungen verbessert, weil mindestens eine Öffnung durch einen sich ausgehend von einem bahnförmigen Element in Richtung auf die gegenüberliegende innere Begrenzungsfläche des anderen bahnförmigen Elements erstreckenden und in den Drainageraum mündenden Kanal gebildet ist, dessen Kanalwand vorzugsweise einstückig mit dem bahnförmigen Element ausgeführt, insbes. durch Perforation des bahnförmigen Elements, gebildet ist.

Im Rahmen der Erfindung kann das Exsudatmanagement besonders wirkungsvoll verbessert werden, wenn sich die Querschnittsfläche des Kanals in einer sich senkrecht zur Tiefenrichtung erstreckenden Ebene ausgehend von dem bahnförmigen Element in Richtung auf die gegenüberliegende innere Begrenzungsfläche, insbes. zum Erhalt einer den Eintritt von Körperflüssigkeiten in den Drainageraum begünstigenden kapillaren Wirkung, verringert. Auf diese Art und Weise wird einerseits der Abtransport von Exsudat aus dem Wundbereich unterstützt und andererseits ein Zurückfließen aus dem Drainageraum in den Wundraum verhindert.

Im Hinblick auf die gewünschte Gesamtstabilität erfindungsgemäßer Wundabdeckungen ist erfindungsgemäß vorgesehen, dass die Mündungen der in einem der bahnförmigen Elemente angeordneten Öffnungen in einer Projektion längs der Tiefenrichtung zwischen den Mündungen der in dem anderen bahnförmigen Element angeordneten Öffnungen angeordnet sind.

Diese Anordnung der Öffnungen in erfindungsgemäßen Wundabdeckungen kann mit besonderem Vorteil bei durch Kanäle gebildeten Öffnungen eingesetzt werden, wenn die Wundabdeckung in Verbindung mit einer zum Absaugen des Exsudats aus dem Wundbereich, insbes. Bauchraum, eingesetzten Unterdruckquelle verwendet wird, um so ein Kollabieren der Gesamtstruktur bei Anlegen von Unterdruck zu verhindern. In diesem Zusammenhang ist erfindungsgemäß vorgesehen, dass sich mindestens ein eine Öffnung in einem bahnförmigen Element bildender Kanal in Tiefenrichtung über 50 % oder mehr der gesamten Tiefe des Drainageraums erstreckt.

Zur Sicherstellung der gewünschten Durchlässigkeit der bahnförmigen Elemente hat es sich als zweckmäßig erwiesen, wenn die dem Drainageraum zugewandte Mündungsfläche der einzelnen Öffnungen in einer senkrecht zur Tiefenrichtung verlaufenden Ebene 0,1 mm² oder mehr, insbes. 0,5 mm² oder mehr, besonders bevorzugt 1 mm² oder mehr, beträgt. Die gewünschte Kapillarwirkung wird erfindungsgemäß unter Vermeidung eines Rückflusses von Flüssigkeit aus dem Drainageraum in den Wundraum erreicht, weil die Mündungsfläche der Öffnungen 5 mm² oder weniger, insbes. 4 mm² oder weniger, besonders bevorzugt 3 mm² oder weniger, beträgt.

Wie vorstehend bereits erläutert, können die die Öffnungen in den bahnförmige Elemente bildenden Kanäle durch Perforationen in den bahnförmigen Elementen gebildet sein. In diesem Zusammenhang hat es sich zum Erhalt einer glatten Oberfläche, mit der ein Anhaften von Gewebe bzw. Zellen am Wundgrund bzw. bei den umliegenden Organen wirkungsvoll unterdrückt werden kann, als günstig erwiesen, wenn die Kanalwand in einer sich parallel zur Tiefenrichtung erstreckenden Schnittebene zumindest abschnittweise bogenförmig ausgeführt ist und stetig in die Begrenzungsfläche des bahnförmigen Elements übergeht. Die Verbindung der bahnförmigen Elemente erfolgt erfindungsgemäß unter gleichzeitiger Sicherstellung des die Drainagewirkung hervorbringenden Drainageraums über punktförmige und vorzugsweise in einer Rasteranordnung ausgebildete Befestigungsbereiche erfolgen. Die Befestigung kann dabei mittels Schweißen, Kleben oder anderen festen Verbindungsarten geschehen. Dabei sollte durch die Verbindung der Abtransport von Exsudaten jedoch nicht behindert, sondern unterstützt werden. In diesem Zusammenhang hat es sich als günstig erwiesen, wenn die einzelnen Befestigungsbereiche in einer sich senkrecht zur Tiefenrichtung erstreckenden Schnittebene eine Fläche von 5 mm² oder weniger, insbes. 3 mm² oder weniger, besonders bevorzugt 2 mm² oder weniger, aufweisen, wobei der Abstand zwischen einzelnen Befestigungsbereichen bzw. einzelnen Rasterpunkten der in einer Rasteranordnung ausgeführten Befestigungsbereiche 2 mm oder mehr, im besonderen 3 mm oder mehr, besonders bevorzugt 5 mm oder mehr, beträgt.

Unabhängig davon, ob die Befestigung durch Schweißen, Kleben oder andere Verbindungsarten erfolgt, kann in den Befestigungsbereichen eine die inneren Begrenzungsflächen der bahnförmigen Elemente miteinander verbindenden Materialbrücke gebildet sein. Zum Erhalt der gewünschten Kapillarwirkung im Drainageraum beträgt der Abstand zwischen den inneren Begrenzungsflächen der bahnförmigen Elemente bei erfindungsgemäßen Wundabdeckungen 5 mm oder weniger, vorzugsweise 4 mm oder weniger, besonders bevorzugt 2 mm oder weniger. Dabei kann die Verteilung der Exsudate über die Gesamtfläche der Wundabdeckung unter Ausnutzung der Kapillarwirkung im Drainageraum sichergestellt werden, wenn der Abstand zwischen den inneren Begrenzungsflächen der bahnförmigen Elemente 0,05 mm oder mehr, insbes. 0,1 mm oder mehr, besonders bevorzugt 0,3 mm oder mehr, beträgt.

Bei erfindungsgemäßen Wundabdeckungen weist mindestens ein bahnförmiges Element eine vorzugsweise perforierte Kunststofffolie auf und ist insbes. insgesamt in Form einer perforierten Kunststofffolie gebildet.

Die erfindungsgemäße Wundabdeckung ist mit besonderem Vorteil in Kombination mit der Unterdrucktherapie zur Wundversorgung einsetzbar. Dabei wird die erfindungsgemäße Wundabdeckung als Wundauflage bzw. als direktes Interface zwischen dem Wundgrund (Gewebe, Zellen) und dem Wundfüller bzw. dem Bereich des Exsudatabtransports eingesetzt. Mit besonderem Vorteil ist die erfindungsgemäße Wundabdeckung für den Einsatz bei Wunden im offenen Abdomen geeignet. Dabei wird die erfindungsgemäße Wundabdeckung in dem gesamten Bauchraum über die Organe (Eingeweide) gelegt, wobei die Wundabdeckung eine Drainagefunktion und eine sehr glatte Oberfläche bietet, um ein Anhaften bzw. eine Friktion von Gewebe und Folie zu verhindern. Die erfindungsgemäße Wundabdeckung kann anatomisch vorgeformt, aber auch auf spezielle Gegebenheiten zuschneidbar ausgeführt sein. Auf die erfindungsgemäße Wundabdeckung wird bei Einsatz in Kombination mit der Unterdrucktherapie ein Füllmedium wie Gaze oder Schaum aufgefüllt, bis der Bereich bis zur Hautgrenze abgedeckt ist. In diesen Wundfüller kann eine ggf. schlauchförmige Wunddrainage eingelegt werden. Der zur Ableitung der Exsudate bzw. zur Wunddrainage eingesetzte Schlauch wird aus dem Bauchraum herausgeführt und an eine Unterdruckquelle angeschlossen, die durch eine Pumpe bzw. im Krankenhaus durch eine zentrale Hausversorgung sichergestellt werden kann. Abschließend wird die Wunde okklusiv mit einer adhäsiven Folie abgedichtet. Mit der Unterdruckquelle kann dann das Exsudat aus dem gesamten Bauchraum mittels der erfindungsgemäßen Wundabdeckung abtransportiert werden, wodurch ein gutes Exsudatmanagement erreicht wird. Die Folie für die okklusive Abdeckung der Wunde ist für die Abschirmung der inneren Organe von der Außenwelt verantwortlich und regelt Feuchtigkeit und Temperatur. Ferner ist sie eine mit der Haut vergleichbare Barriere gegen Bakterien, Viren und Keime.

Ein Verfahren zur Herstellung erfindungsgemäßer Wundabdeckungen ist im wesentlichen dadurch gekennzeichnet, daß ein bahnförmiges Material, bspw. unter Verwendung einer Stanzwalze, zur Bildung trichterförmiger Öffnungen perforiert wird, zwei perforierte Abschnitte des bahnförmigen Materials mit einander zugewandten Trichteröffnungen übereinandergelegt und anschließend bspw. mittels eines Punktschweißverfahrens miteinander verbunden werden, wie genau definiert in Anspruch 11.

Bei diesem Verfahren kann der gewünschte Abstand zwischen den inneren Begrenzungsflächen durch die Tiefe der trichterförmigen Öffnungen sichergestellt werden. Dabei kann der Perforationsvorgang allerdings nur mit begrenzter Genauigkeit erfolgen, so daß selbst dann, wenn einige Trichteröffnungen an der gegenüberliegenden inneren Begrenzungsfläche des gegenüberliegenden bahnförmigen Elements anliegen, andere Trichteröffnungen noch frei in den Drainageraum münden.

Im Hinblick auf die Vermeidung der Verklebungsneigung erfindungsgemäßer Wundabdeckungen kann mindestens eine Materialbahn eine hydrophile oder hydrophobe Ausrüstung aufweisen. Ferner ist auch an den Auftrag von quellbaren Materialen gedacht.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: eine schematische Darstellung einer erfindungsgemäßen Wundabdeckung und
- **Fig. 2**: ein Anwendungsbeispiel

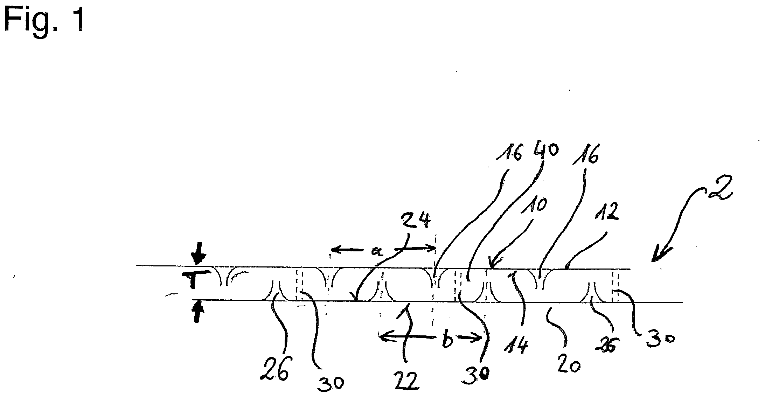

Die in Fig. 1 dargestellte Wundabdeckung umfaßt ein erstes, eine äußere Begrenzungsfläche 12 aufweisendes, bahnförmiges Element 10 sowie ein zweites, eine äußere Begrenzungsfläche 22 aufweisendes, bahnförmiges Element 20. Zwischen einer dem zweiten bahnförmigen Element 20 zugewandten inneren Begrenzungsfläche 14 des ersten bahnförmigen Elements 10 und einer dem ersten bahnförmigen Element 10 zugewandten inneren Begrenzungsfläche 24 des zweiten bahnförmigen Elements 20 ist ein Drainageraum 40 gebildet. Die Tiefe des Drainageraums 40 in einer senkrecht zu den Hauptflächen der bahnförmigen Elemente 10 und 20 verlaufenden Richtung beträgt bei der in der Zeichnung dargestellten Ausführungsform der Erfindung zwischen 0,1 und 4 mm.

Das erste bahnförmige Element 10 weist eine Mehrzahl von rasterförmig angeordneten, kanalförmigen Perforationen 16 auf. Die Kanalwände der Kanäle 16 sind in einer parallel zur Tiefenrichtung verlaufenden Schnittebene bogenförmig ausgeführt und gehen glatt in die äußere Begrenzungsfläche 12 des ersten bahnförmigen Elements 10 über. Ebenso weist das zweite bahnförmige Element 20 eine Anzahl von rasterförmig angeordneten, ebenfalls kanalförmigen Perforationen 26 auf. Die Kanäle 16 und 26 erstrecken sich jeweils über mehr als die Hälfte der Gesamttiefe T des Drainageraums 40. Dabei sind die Kanäle 12 und 16 derart angeordnet, daß eine Projektion der Kanalmündungen der Kanäle 16 in der Tiefenrichtung zwischen den Kanalmündungen der Kanäle 26 zu liegen kommt.

Im Mündungsbereich weisen die bei der anhand der Zeichnung erläuterten Ausführungsform der Erfindung kegelförmig ausgeführten Kanäle 16 bzw. 26 einen Durchmesser von 0,2 bis 1 mm auf. Die bahnförmigen Elemente 10 und 20 sind über rasterförmig angeordnete Materialbrücken 30 miteinander verbunden. Durch die Materialbrücken 30 wird der Abstand zwischen den inneren Begrenzungsflächen 14 und 24 eingestellt. Die Verbindungsbereiche 30 können durch Schweißen, Kleben oder andere feste Verbindungsarten verwirklicht werden. Die bahnförmigen Elemente 10 und 20 können in Form von Kunststofffolien verwirklicht werden. Mindestens ein bahnförmiges Element (10, 20) weist eine vorzugsweise perforierte Kunststofffolie auf.

In diesem Zusammenhang können sowohl synthetische Kunststoffe, wie etwa PE, PP, PET, Polyurethan (PUR), Teflon (PTFE), als auch Folien auf Basis biologischer Kunststoffe bzw. nachwachsender Kunststoffe, wie etwa Polyhydroxybutyrat (PHB), Polylactat (PLA), Polymilchsäure, Rohstoffe auf Basis von Cellulose (CMC) usw. zum Einsatz kommen. Die Struktur der perforierten Materialbahnen kann mittels eines non woven Herstellungsprozesses erzeugt werden. Dabei können zur Herstellung der non wovens oder auch Gewebe synthetische wie auch natürliche (z. B. Seide, Baumwolle) Fasern, wie auch anorganische Fasern (Glaskeramik...) bzw. Metall (Silberfasern) verwendet werden. Im Rahmen der Erfindung ist ferner daran gedacht, daß die Materialbahnen 10, 20 bzw. der Drainageraum 40 mit einer antibakteriellen bzw. bakteriostatischen Schicht ausgestattet werden. Die antibakterielle bzw. bakertiostatische Wirkung kann bspw. durch PHMB, Silber, Chlorhexidin usw. verwirklicht werden.

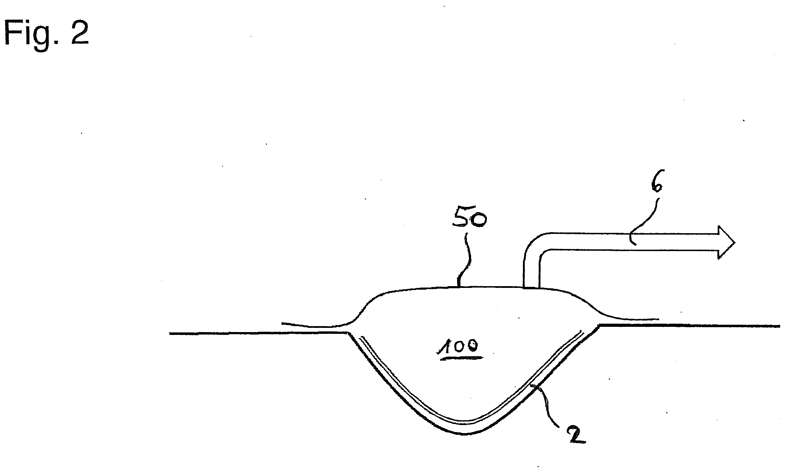

Gemäß dem anhand der Figur 2 erläuterten Anwendungsbeispiel wird die Wundabdeckung 2 auf dem Wundgrund aufgelegt, die Wunde danach mit einem Füllmaterial, wie etwa einem Schaum, Gaze od. dgl., aufgefüllt und mit einer okklusiven Folie 50 abgedeckt. Die okklusive Folie 50 wird von einer Exsudatleitung 6 durchsetzt, welche zum Absaugen des Exsudats aus dem Wundraum an eine Unterdruckquelle angeschlossen werden kann.

Bei Einsatz erfindungsgemäßer Wundabdeckungen ergeben sich, wie vorstehend erläutert, die folgenden Vorteile:
- Die Poren mit deren nach innen sich erhebenden Struktur unterstützen den Abtransport von Exsudat und verhindern das Zurückfließen des Exsudats in den Wundraum (Kapillarwirkung).
- Die offenen Drainagekammern geben der Drainageschicht 40 eine kapillare Wirkung und lassen somit kein Abfließen aus der Wundabdeckung zu.
- Die durch den glatten Übergang zwischen den Kanälen 16 bzw. 26 und den äußeren Begrenzungsflächen 12 bzw. 24 der bahnförmigen Elemente erhaltene glatte Oberfläche verhindert ein Anhaften von Gewebe bzw. Zellen am Wundgrund bzw. bei den umliegenden Organen.
- Die Kombination aus Porengröße und Glattheit der Folie verhindert ein Einwachsen bzw. Anhaften von Gewebe.
- Der Drainageraum 40 bietet immer einen offenen Raum für den Abtransport von Exsudat.
- Die Wundabdeckung ist ohne Beeinträchtigung der Drainagefunktion auf jede Grö-ße bzw. Struktur im Wund- bzw. Abdominalraum zuschneidbar.

Abschließend wird darauf hingewiesen, daß der Abstand zwischen den Zentrumsachsen der Kanäle 16 bzw. 26, wie in Figur 1 bei a und b angedeutet, der anhand der Zeichnung erläuterten Ausführungsform der Erfindung mindestens 0,1 mm und maximal 4 mm beträgt.

## Patentansprüche

1. Wundabdeckung mit einem eine erste Begrenzungsfläche (12) bildenden ersten bahnförmigen Element (10) und einem eine der ersten Begrenzungsfläche (12) abgewandte und etwa parallel dazu verlaufende zweite Begrenzungsfläche (22) bildenden und mit dem ersten bahnförmigen Element (10) verbundenen zweiten bahnförmigen Element (20), wobei zwischen der der ersten Begrenzungsfläche (12) abgewandten ersten inneren Begrenzungsfläche (14) des ersten bahnförmigen Elements (10) und einer der zweiten Begrenzungsfläche (22) abgewandten zweiten inneren Begrenzungsfläche (24) des zweiten bahnförmigen Elements (20) mindestens ein Drainageraum (40) gebildet ist, dessen Tiefe (T) in einer sich senkrecht zu den Begrenzungsflächen (12, 22) erstreckenden Tiefenrichtung eine Kapillarwirkung auf in dem Drainageraum (40) aufgenommene Körperflüssigkeiten, insbes. Exsudate, gewährleistet, wobei mindestens eines der bahnförmigen Elemente (10, 20) eine den Durchtritt von Körperflüssigkeiten in den Drainageraum (40) ermöglichende Öffnung aufweist, welche Öffnung durch einen sich ausgehend von einem bahnförmigen Element (10, 20) in Richtung auf die gegenüberliegende innere Begrenzungsfläche (14, 24) des anderen bahnförmigen Elements (10, 20) erstreckenden und in den Drainageraum (40) mündenden Kanal (16, 26) gebildet ist, dessen Kanalwand vorzugsweise einstückig mit dem bahnförmigen Element (10, 20) ausgeführt, insbes. durch Perforation des bahnförmigen Elements (10, 20), gebildet ist, wobei die Mündungen der in einem bahnförmigen Element (10, 20) angeordneten Öffnungen in einer Projektion längs der Tiefenrichtung zwischen den Mündungen der in dem anderen bahnförmigen Element (10, 20) angeordneten Öffnungen angeordnet sind, wobei sich mindestens ein Kanal (16, 26) in Tiefenrichtung über 50 % oder mehr der gesamten Tiefe (T) des Drainageraums (40) erstreckt, **dadurch gekennzeichnet, dass** die dem Drainageraum (40) zugewandte Mündungsfläche der einzelnen Öffnungen in einer senkrecht zur Tiefenrichtung verlaufenden Ebene 5 mm² oder weniger, insbes. 4 mm² oder weniger, besonders bevorzugt 3 mm² oder weniger, beträgt, die bahnförmigen Elemente (10, 20) über punktförmige und vorzugsweise in einer Rasteranordnung ausgeführte Befestigungsbereiche (30) miteinander verbunden sind und mindestens ein bahnförmiges Element (10, 20) eine vorzugsweise perforierte Kunststofffolie aufweist.

2. Wundabdeckung nach Anspruch 1, **dadurch gekennzeichnet, daß** sich die Querschnittsfläche des Kanals (16, 26) in einer sich senkrecht zur Tiefenrichtung erstreckenden Ebene ausgehend von dem bahnförmigen Element (10, 20) in Richtung auf die gegenüberliegende andere Begrenzungsfläche (14, 24), insbes. zum Erhalt einer den Eintritt von Körperflüssigkeiten in den Drainageraum (40) begünstigenden Kapillarwirkung, verringert.

3. Wundabdeckung nach einem der vorhergehenden Ansprüche, **dadurch gekennzeichnet, daß** die dem Drainageraum (40) zugewandte Mündungsfläche der einzelnen Öffnungen in einer senkrecht zur Tiefenrichtung verlaufenden Ebene 0,1 mm² oder mehr, insbes. 0,5 mm² oder mehr, besonders bevorzugt 1 mm² oder mehr, beträgt.

4. Wundabdeckung nach Anspruch 2 oder 3, **dadurch gekennzeichnet, daß** die Kanalwand in einer sich parallel zur Tiefenrichtung erstreckenden Schnittebene zumindest abschnittweise bogenförmig ausgeführt ist und stetig in die Begrenzungsfläche (12, 22) übergeht.

5. Wundabdeckung nach einem der vorhergehenden Ansprüche, **dadurch gekennzeichnet, daß** die einzelnen Befestigungsbereiche (30) in einer sich senkrecht zur Tiefenrichtung erstreckenden Schnittebene eine Fläche von 5 mm² oder weniger, insbes. 3 mm² oder weniger, besonders bevorzugt 2 mm² oder weniger, aufweisen.

6. Wundabdeckung nach einem der vorhergehenden Ansprüche, **dadurch gekennzeichnet, daß** in den Befestigungsbereichen (30) eine die inneren Begrenzungsflächen (14, 24) der Materialbahnen (10, 20) verbindende Materialbrücke gebildet ist.

7. Wundabdeckung nach einem der vorhergehenden Ansprüche, **dadurch gekennzeichnet, daß** der Abstand zwischen den inneren Begrenzungsflächen (14, 24) 5 mm oder weniger, insbes. 4 mm oder weniger, besonders bevorzugt 2 mm oder weniger, beträgt.

8. Wundabdeckung nach einem der vorhergehenden Ansprüche, **dadurch gekennzeichnet, daß** der Abstand zwischen den inneren Begrenzungsflächen (14, 24) 0,05 mm oder mehr, insbes. 0,1 mm oder mehr, besonders bevorzugt 0,3 mm oder mehr, beträgt.

9. Wundabdeckung nach einem der vorhergehenden Ansprüche, **dadurch gekennzeichnet, daß** mindestens ein bahnförmiges Element (10, 20) ein antibakterielles bzw. bakteriostatisches Material, wie etwa PHMB, Silber, Chlorhexidin od. dgl., insbes. in Form einer Oberflächenschicht, aufweist.

10. Wundabdeckung nach einem der vorhergehenden Ansprüche, **dadurch gekennzeichnet, daß** mindestens ein bahnförmiges Element ein der Verklebungsneigung der Wundabdeckung entgegenwirkendes Mittel, wie etwa eine hydrophile oder hydrophobe Ausrüstung oder den Auftrag von quellbaren Materialien aufweist.

11. Verfahren zum Herstellen einer Wundabdeckung nach einem der vorhergehenden Ansprüche, bei dem ein bahnförmiges Material bspw. unter Verwendung einer Stanzwalze zur Bildung trichterförmiger Öffnungen perforiert wird, zwei perforierte Abschnitte des bahnförmigen Materials mit einander zugewandten Trichteröffnungen übereinandergelegt und anschließend bspw. mittels eines Punktschweißverfahrens miteinander verbunden werden.

## Claims

1. Wound covering with a first web-type element (10) forming a first boundary surface (12) and a second web-type element (20) forming a second boundary surface (22) facing away from the first boundary surface (12) and running approximately parallel thereto and connected to the first web-shaped element (10), wherein at least one drainage space (40) is formed between the first inner boundary surface (14) of the first web-type element (10) facing away from the first boundary surface (12) and a second inner boundary surface (24) of the second web-type element (20) facing away from the second boundary surface (22), the depth (T) of which drainage space (40) in a depth direction extending perpendicularly to the boundary surfaces (12, 22) has a capillary effect on body fluids, in particular exudates, received in the drainage space (40), wherein at least one of the web-type elements (10, 20) has an opening enabling the passage of body fluids into the drainage space (40), which opening is formed by a channel (16, 26) extending from one web-type element (10, 20) in the direction of the opposite inner boundary surface (14, 24) of the other web-type element (10, 20) and opening out into the drainage space (40), the channel wall of which channel (16, 26) is preferably formed in one piece with the web-type element (10, 20), in particular by perforation of the web-type element (10, 20), wherein the orifices of the openings arranged in one web-type element (10, 20) are arranged in a projection along the depth direction between the orifices of the openings arranged in the other web-type element (10, 20), wherein at least one channel (16, 26) extends in the depth direction over 50% or more of the entire depth (T) of the drainage space (40), **characterised in that** the orifice surface of the individual openings facing the drainage space (40) in a plane running perpendicularly to the depth direction is 5 mm² or less, in particular 4 mm² or less, particularly preferably 3 mm² or less, the web-type elements (10, 20) are connected to one another via punctiform fastening regions (30) preferably formed in a grid arrangement and at least one web-type element (10, 20) has a preferably perforated plastic film.

2. Wound covering according to claim 1, **characterised in that** the cross-sectional area of the channel (16, 26) in a plane extending perpendicularly to the depth direction decreases from the web-type element (10, 20) in the direction of the opposite other boundary surface (14, 24), in particular to obtain a capillary effect promoting the entry of body fluids into the drainage space (40).

3. Wound covering according to one of the preceding claims, **characterised in that** the mouth area of the individual openings facing the drainage space (40) in a plane running perpendicularly to the depth direction is 0.1 mm² or more, in particular 0.5 mm² or more, particularly preferably 1 mm² or more.

4. Wound covering according to claim 2 or 3, **characterised in that** the channel wall in a cutting plane extending parallel to the depth direction is of arcuate design at least in sections and merges continuously into the boundary surface (12, 22).

5. Wound covering according to one of the preceding claims, **characterised in that** the individual fastening regions (30) in a cutting plane extending perpendicularly to the depth direction have an area of 5 mm² or less, in particular 3 mm² or less, particularly preferably 2 mm² or less.

6. Wound covering according to one of the preceding claims, **characterised in that** a material bridge connecting the inner boundary surfaces (14, 24) of the material webs (10, 20) is formed in the fastening regions (30).

7. Wound covering according to one of the preceding claims, **characterised in that** the distance between the inner boundary surfaces (14, 24) is 5 mm or less, in particular 4 mm or less, particularly preferably 2 mm or less.

8. Wound covering according to one of the preceding claims, **characterised in that** the distance between the inner boundary surfaces (14, 24) is 0.05 mm or more, in particular 0.1 mm or more, particularly preferably 0.3 mm or more.

9. Wound covering according to one of the preceding claims, **characterised in that** at least one web-shaped element (10, 20) has an antibacterial or bacteriostatic material, such as, for instance, PHMB, silver, chlorhexidine or the like, in particular in the form of a surface layer.

10. Wound covering according to one of the preceding claims, **characterised in that** at least one web-shaped element has an agent counteracting the tendency of the wound covering to stick, such as, for instance, a hydrophilic or hydrophobic finish or the application of swellable materials.

11. Method for producing a wound covering according to one of the preceding claims, in which a web-shaped material is perforated, for example using a punching roller, to form funnel-shaped openings, two perforated sections of the web-shaped material with funnel openings facing one another are placed one above the other and are then connected to one another, for example by means of a spot welding method.

## Revendications

1. Pansement avec un premier élément en forme de bande (10) formant une première surface de délimitation (12) et un deuxième élément en forme de bande (20) et relié au premier élément en forme de bande (10) et formant une deuxième surface de délimitation (22) opposée à la première surface de délimitation (12) et s'étendant sensiblement parallèlement à celle-ci, dans lequel au moins un espace de drainage (40) est formé entre la première surface de délimitation intérieure (14) du premier élément en forme de bande (10) opposée à la première surface de délimitation (12) et une deuxième surface de délimitation intérieure (24) du deuxième élément en forme de bande (20) opposée à la deuxième surface de délimitation (22), dont la profondeur (T) dans une direction de profondeur s'étendant perpendiculairement aux surfaces de délimitation (12, 22) assure un effet capillaire sur des fluides corporels, notamment des liquides corporels, notamment des exsudats, reçus dans l'espace de drainage (40), dans lequel au moins l'un des éléments en forme de bande (10, 20) présente une ouverture permettant le passage de fluides corporels dans l'espace de drainage (40), laquelle ouverture est formée par un canal (16, 26) s'étendant à partir d'un élément en forme de bande (10, 20) en direction de la surface de délimitation intérieure opposée (14, 24) de l'autre élément en forme de bande (10, 20) et débouchant dans l'espace de drainage (40), dont la paroi de canal est réalisée de préférence d'une seule pièce avec l'élément en forme de bande (10, 20), notamment par perforation de l'élément en forme de bande (10, 20), dans lequel les embouchures des ouvertures disposées dans un élément en forme de bande (10, 20) sont disposées dans une projection le long de la direction de profondeur entre les embouchures des ouvertures disposées dans l'autre élément en forme de bande (10, 20), dans lequel au moins un canal (16, 26) s'étend dans la direction de profondeur sur 50 % ou plus de la profondeur totale (T) de l'espace de drainage (40), **caractérisé en ce que** la superficie d'embouchure tournée vers l'espace de drainage (40) des ouvertures individuelles dans un plan s'étendant perpendiculairement à la direction de profondeur est de 5 mm² ou moins, notamment de 4 mm² ou moins, de manière particulièrement préférée de 3 mm² ou moins, les éléments en forme de bande (10, 20) sont reliés l'un à l'autre par des zones de fixation (30) en forme de points et réalisées de préférence dans un agencement en grille et au moins un élément en forme de bande (10, 20) présente un film plastique de préférence perforé.

2. Pansement selon la revendication 1, **caractérisé en ce que** la surface de section transversale du canal (16, 26) dans un plan s'étendant perpendiculairement à la direction de profondeur diminue à partir de l'élément en forme de bande (10, 20) en direction de l'autre surface de délimitation opposée (14, 24), notamment pour obtenir un effet capillaire favorisant l'entrée de fluides corporels dans l'espace de drainage (40).

3. Pansement selon l'une quelconque des revendications précédentes, **caractérisé en ce que** la superficie d'embouchure tournée vers l'espace de drainage (40) des ouvertures individuelles dans un plan s'étendant perpendiculairement à la direction de profondeur est de 0,1 mm² ou plus, notamment de 0,5 mm² ou plus, de manière particulièrement préférée de 1 mm² ou plus.

4. Pansement selon la revendication 2 ou 3, **caractérisé en ce que** la paroi de canal est réalisée au moins en partie en forme d'arc dans un plan de coupe s'étendant parallèlement à la direction de profondeur et se prolonge en continu par la surface de délimitation (12, 22).

5. Pansement selon l'une quelconque des revendications précédentes, **caractérisé en ce que** les zones de fixation individuelles (30) présentent une surface de 5 mm² ou moins, notamment de 3 mm² ou moins, de manière particulièrement préférée de 2 mm² ou moins dans un plan de coupe s'étendant perpendiculairement à la direction de profondeur.

6. Pansement selon l'une quelconque des revendications précédentes, **caractérisé en ce qu'**un pont de matériau reliant les surfaces de délimitation intérieures (14, 24) des bandes de matériau (10, 20) est formé dans les zones de fixation (30).

7. Pansement selon l'une quelconque des revendications précédentes, **caractérisé en ce que** la distance entre les surfaces de délimitation intérieures (14, 24) est de 5 mm ou moins, notamment de 4 mm ou moins, de manière particulièrement préférée de 2 mm ou moins.

8. Pansement selon l'une quelconque des revendications précédentes, **caractérisé en ce que** la distance entre les surfaces de délimitation intérieures (14, 24) est de 0,05 mm ou plus, notamment de 0,1 mm ou plus, de manière particulièrement préférée de 0,3 mm ou plus.

9. Pansement selon l'une quelconque des revendications précédentes, **caractérisé en ce qu'**au moins un élément en forme de bande (10, 20) présente un matériau antibactérien ou bactériostatique, tel que le PHMB, l'argent, la chlorhexidine ou similaire, notamment sous la forme d'une couche de superficie.

10. Pansement selon l'une quelconque des revendications précédentes, **caractérisé en ce qu'**au moins un élément en forme de bande présente un agent inhibant la tendance du pansement à l'adhérence, tel qu'un équipement hydrophile ou hydrophobe ou l'application de matériaux gonflables.

11. Procédé de fabrication d'un pansement selon l'une quelconque des revendications précédentes, dans lequel un matériau en forme de bande est perforé par exemple en utilisant un cylindre de poinçonnage pour former des ouvertures en forme d'entonnoir, deux sections perforées du matériau en forme de bande sont superposées avec des ouvertures d'entonnoir tournées l'une vers l'autre et ensuite reliées l'une à l'autre par exemple au moyen d'un procédé de soudage par points.
